# FASCICULE DE BREVET EUROPEEN

(11) **EP 1 242 375 B1**
(45) Date de publication et mention de la délivrance du brevet: **12.05.2004**
(21) Numéro de dépôt: 00993712.9
(22) Date de dépôt: 27.12.2000
(51) Int. Cl.: C07D 209/16

(54) **PROCEDE DE PURIFICATION DE LACTAMES**
VERFAHREN ZUR REINIGUNG VON LACTAMEN
METHOD FOR PURIFYING LACTAMES

(30) Priorité: 30.12.1999 FR 9916713
(43) Date de publication de la demande: 25.09.2002
(73) Titulaire: Rhodia Polyamide Intermediates, 69192 Saint-Fons (FR)
(72) Inventeur: CARVIN, Philippe, F-69005 Lyon (FR); MASSON, Jean-Claude, F-69003 Lyon (FR)
(74) Mandataire: Esson, Jean-Pierre
(86) Numéro de dépôt international: PCT/FR2000/003694
(87) Numéro de publication internationale: WO 2001/049665

(56) Documents cités:
- EP-A- 0 943 608
- FR-A- 2 751 962
- US-A- 5 496 941

## Description

L'invention concerne un procédé de purification de lactames, notamment les lactames obtenus par hydrolyse cyclisante d'un aminoalkylnitrile.

Elle se rapporte plus particulièrement à la purification de l'ε-caprolactame obtenu par hydrolyse cyclisante de l'aminocapronitrile.

Les lactames et notamment l'ε-caprolactame sont des composés chimiques importants utilisés comme matières premières dans la fabrication de nombreux produits. Une des plus importantes applications est la fabrication de polymères tels que les polyamides.

Parmi les procédés de synthèse des lactames et notamment de l'ε-caprolactame, il a été proposé un procédé consistant à fabriquer un aminoalkylnitrile tel que l'aminocapronitrile puis à réaliser une hydrolyse cyclisante de cet aminonitrile. Ce procédé qui peut être mis en oeuvre en milieu liquide ou gazeux avec ou sans solvant est décrit par exemple dans les brevets Ep 0729454, Ep 0729453, Ep 0793650, Ep 0793651, Ep 0794943, Ep 0815077, Ep 0815078, Ep 748797, Ep 659741, WO 96/22974.

Une étape importante dans la fabrication des lactames, notamment de l'ε-caprolactame quand ceux-ci doivent être utilisés comme monomères dans la fabrication de polymères tels que polyamides, est la purification des produits. En effet, pour ces applications, le lactame doit satisfaire à des critères de pureté très contraignants et élevés. Ces critères sont définis par différents indices ou concentrations correspondant à plusieurs types ou catégories d'impuretés. Ces critères principaux sont :
Indice permanganate (Ik_{MnO4})
Teneur en bases volatiles (I_{Bv})
Indice UV (I_{UV})

Différents procédés de purification ont déjà été proposés. Ainsi, le brevet US 5,496,941 décrit un procédé de purification de caprolactame extrait du milieu réactionnel d'hydrolyse cyclisante par distillation comprenant une étape d'hydrogénation suivie par un traitement en milieu acide (traitement sur résine échangeuse d'ions ou distillation en milieu acide sulfurique) et une distillation en milieu basique.

La demande de brevet européen n° 943608 décrit un procédé de purification de l'ε-caprolactame brut obtenu par cyclisation d'un alkyl 6-aminocaproate consistant à effectuer une cristallisation du caprolactame. D'après ce document, certains composants comme le N-méthyl caprolactame, le méthyl-valérolactame et le valéramide sont éliminés par cristallisation. Le procédé décrit indique que le caprolactame ainsi cristallisé respecte les critères requis de pureté concernant la teneur en bases volatiles et l'indice UV d'absorption à une longueur d'onde de 290nm.

Toutefois, ce document ne précise pas si le troisième critère de pureté, l'indice permanganate est atteint.

Dans le cas d'un caprolactame issu de l'hydrolyse cyclisante d'un aminocapronitrile obtenu par hémihydrogénation de l'adiponitrile, les critères de pureté du caprolactame sont atteints par mise en oeuvre des procédés de purification décrits ci-dessus comprenant plusieurs traitements successifs, notamment un traitement acide et une distillation finale en milieu basique.

Dans de tels procédés l'obtention d'un caprolactame de pureté requise demande la réalisation d'une distillation avec une fraction de lourds (fraction des produits à haut point d'ébullition) importante comportant une quantité élevée de lactame sous forme lactame ou oligomères. Cette perte en lactame est préjudiciable au procédé.

Un des buts de la présente invention est de remédier notamment à cet inconvénient en proposant un procédé de purification de lactames permettant d'obtenir un produit satisfaisant aux critères de pureté requis limitant les étapes de traitement du lactame et plus particulièrement celles pouvant générer la formation d'oligomères.

A cet effet, l'invention propose un procédé de purification d'un lactame issu de la cyclisation d'un aminoalkylnitrile obtenu par hémihydrogénation d'un alkyledinitrile consistant à soumettre à une hydrogénation le milieu de cyclisation constitué par le milieu réactionnel issu de la réaction de cyclisation, à éliminer les composés volatiles et les composés à point d'ébullition élevé, à traiter ledit milieu par une résine échangeuse d'ions et à récupérer le lactame par cristallisation a.

Selon une autre caractéristique préférentielle de l'invention, le lactame cristallisé peut être soumis à une ou plusieurs autres étapes de cristallisation pour obtenir la pureté requise.

Par simplification le terme "milieu de cyclisation" pour la mise en oeuvre de la cristallisation signifie soit le milieu réactionnel issu de l'étape de cyclisation sur lequel sont réalisés les différents traitements décrits ci-dessus, soit le milieu formé par le caprolactame extrait après la réaction de cyclisation, par exemple par distillation ou entraînement, en d'autres termes après élimination ou séparation des composés volatils et des composés à point d'ébullition élevé. Le classement en composés volatils et composés à point d'ébullition élevé se fait par rapport au point d'ébullition du lactame à récupérer.

Selon l'invention, l'extraction et la récupération du lactame par une cristallisation permettent d'obtenir un produit répondant aux critères de pureté requis, sans nécessité de traitement à des températures pouvant générer la formation d'oligomères, par exemple.

Ainsi la dégradation ou polymérisation du lactame qui intervient quand il est chauffé à des températures élevées, notamment pour permettre sa distillation, est évitée.

Le procédé de l'invention s'applique plus particulièrement à la purification de l'ε-caprolactame obtenu par hydrolyse cyclisante de l'aminocapronitrile.

Cet aminocapronitrile est synthétisé par hémihydrogénation de l'adiponitrile. Ces différents procédés d'hémihydrogénation sont décrits, par exemple, dans les brevets Ep 737100, Ep 737181, WO96/18603.

Selon l'invention, les solvants convenables pour la cristallisation peuvent être un lactame, l'eau, les hydrocarbures saturés tels que l'hexane ou le cyclohexane, les alcools, les hydrocarbures aromatiques, les composés organiques halogènés comme CCl₄, CHCl₃, les cétones ou analogues.

Comme solvants préférés, on utilisera l'eau et/ou un lactame et plus préférentiellement un lactame identique à celui qui doit être purifié ou encore plus préférentiellement une solution aqueuse saturée en lactame.

La cristallisation peut être réalisée en une seule étape ou en plusieurs étapes. La liqueur mère obtenue à une étape est avantageusement recyclée dans une des étapes précédentes. De manière plus générale, la cristallisation peut être mise en oeuvre selon les techniques classiques et industrielles de cristallisation.

Selon une autre caractéristique du procédé de l'invention, le composé obtenu après cristallisation est filtré, essoré ou isolé par toute autre technologie usuelle de séparation solide/liquide.Le solide récupéré peut être avantageusement lavé avec un solvant, de préférence avec une solution saturée en lactame.

Avantageusement, le milieu contenant le lactame à cristalliser est concentré par tout moyen approprié pour obtenir une concentration pondérale en lactame supérieur à 80 % en poids, de préférence supérieure à 90%.

Ainsi, on peut citer comme exemple de techniques de saturation ou concentration de la solution, les techniques de refroidissement, évaporation avantageusement sous pression réduite pour travailler à plus basse température.

En outre, la cristallisation peut être réalisée avec un ensemencement de la solution pour favoriser la nucléation du lactame.

Le lactame pur récupéré après séparation solide/liquide, par exemple filtration, et lavage éventuel, est séché ou distillation du solvant, par exemple l'eau, selon des techniques usuelles (quand le solvant est l'eau, le caprolactame est deshydraté).

Le procédé de l'invention permet donc de produire un lactame, plus particulièrement un ε-caprolactame présentant les critères de pureté requis pour une utilisation comme monomère dans la fabrication des polyamides tels que le PA6. Les polyamides ainsi obtenus sont convenables pour, notamment, la fabrication de fils ou fibres textiles avec des propriétés d'usage équivalentes aux polyamides actuels.

D'autres avantages, détails de l'invention seront illustrés par les exemples de réalisation donnés ci-dessous uniquement a titre indicatif.

### Exemple 1

Un ε-caprolactame est fabriqué selon le procédé décrit dans les brevets cités précédemment.

Ainsi, cet ε-caprolactame est obtenu selon l'exemple 1 du brevet EP 748797 par hémihydrogénation d'adiponitrile puis hydrolyse cyclisante d'aminocapronitrile selon les conditions décrites dans cet exemple.

Le milieu obtenu est soumis à une hydrogénation selon le procédé décrit dans la demande de brevet français déposée sous le n°98 14735 le 19 novembre 1998, non encore publiée.

L'ammoniac présent dans le milieu réactionnel d'hydrolyse cyclisante hydrogéné est ensuite éliminé par évaporation ou entrainement.

Le milieu de cyclisation ainsi obtenu est ensuite traité par passage sur une résine échangeuse d'ions selon les conditions décrites dans les brevets ou demandes de brevet WO98/05636 ou WO96/20923.

Le milieu obtenu après traitement sur résine contient 65% en poids d'ε-caprolactame et de nombreux autres produits qui n'ont pas tous été identifiés.

Le milieu ainsi récupéré est analysé pour déterminer les critère de pureté définis précedemment. Les résultats de ces analyses sont indiqués dans le tableau ci-dessous.

Conformément au procédé de l'invention, le caprolactame est récupéré par cristallisation à partir de cette solution après concentration sous pression réduite à une teneur en caprolactame voisine de 90% en poids.

La solution concentrée est à une température de 40°C puis refroidie jusqu'à une température voisine de 20°C avec une vitesse de refroidissement d'environ 10°C par heure.

Les cristaux formés sont récupérés par filtration sur un fritté. Les eaux mères récupérées sont stockées pour un éventuel recyclage.

Le gâteau solide de cristaux de caprolactame est lavé par une solution aqueuse saturée en caprolactame. Le taux de lavage en base humide est avantageusement compris entre 1 et 5.

Le caprolactame cristallisé est analysé. Les caractéristiques de pureté sont rassemblées dans le tableau I ci-dessous.

| **Essai** | **Milieu** | **I**_{**UV**} | **I**_{**KMO4**} | **I**_{**BV**}**(meq/kg)** |
|---|---|---|---|---|
| | Milieu avant cristallisation | 0,45 | 32,06 | 2,69 |
| | Gâteau humide après lavage | 0,02 | 2,03 | 0,04 |

### Exemple 2

Un caprolactame est fabriqué selon le procédé décrit à l'exemple 1 et en référence au procédé décrit dans la demande de brevet EP 748797.

Toutefois dans cet exemple, le milieu issu de l'hydrolyse cyclisante est uniquement traité pour éliminer les composés volatils, plus particulièrement l'ammoniac.

La cristallisation du caprolactame formé est réalisée directement à partir de cette solution après concentration par évaporation d'eau.

La cristallisation est réalisée en deux étapes successives. Le gâteau récupéré en première étape est repris pour obtenir une nouvelle solution aqueuse saturée puis recristallisé. Les caractéristiques de pureté du gâteau recristallisé après lavage sont indiquées dans le tableau II ci-dessous.

| **Essai** | **Milieu** | **I**_{**UV**} | **I**_{**KMO4**} | **I**_{**BV**} **(meq/kg)** |
|---|---|---|---|---|
| 2 | Milieu avant cristallisation | 17,7 | 400 | 130 |
| | Gâteau humide recristallisé après lavage | 0,03 | 2,0 | 0,20 |

## Revendications

1. Procédé de purification de lactames obtenus par cyclisation d'un aminoalkylnitrile, **caractérisé en ce qu'**il consiste à soumettre à une hydrogénation le milieu de cyclisation constitué par le milieu réactionnel issu de la réaction de cyclisation, à éliminer les composés volatiles et les composés à point d'ébullition élevé, à traiter ledit milieu par une résine échangeuse d'ions et à récupérer le lactame par cristallisation.

2. Procédé selon la revendication 1, **caractérisé en ce que** la cristallisation est réalisée en plusieurs étapes

3. Procédé selon l'une des revendications précédentes **caractérisé en ce que** le lactame est l'ε-caprolactame.

4. Procédé selon la revendication 3 **caractérisé en ce que** l'ε-caprolactame est produit par hydrolyse cyclisante de l'aminocapronitrile.

5. Procédé selon la revendication 4 **caractérisé en ce que** l'aminocapronitrile est synthétisé par hémihydrogénation de l'adiponitrile.

6. Procédé selon l'une des revendications précédentes **caractérisé en ce que** le solvant de cristallisation est choisi dans le groupe comprenant, un lactame, une solution aqueuse de lactame, l'eau; les hydrocarbures saturés, les alcools, les hydrocarbures aromatiques, les composés organiques halogénés, les cétones.

7. Procédé selon la revendication 6 **caractérisé en ce que** le solvant est une solution aqueuse saturée de lactame.

8. Procédé selon l'une des revendications précédentes, **caractérisé en ce que** la concentration pondérale en lactame dans la solution à cristalliser est supérieure à 80% en poids

## Patentansprüche

1. Verfahren zur Reinigung von Lactamen, die erhalten wurden durch Cyclisierung eines Aminoalkylnitrils, **dadurch gekennzeichnet, daß** es darin besteht, das Medium der Cyclisierung, das gebildet wird durch das aus der Cyclisierungsreaktion stammende Reaktionsmedium, einer Hydrierung zu unterziehen, die flüchtigen Verbindungen und die Verbindungen mit hohem Siedepunkt abzutrennen, das genannte Medium durch ein Ionenaustauscherharz zu behandeln und das Lactam durch Kristallisation zu gewinnen.

2. Verfahren nach Anspruch 1, **dadurch gekennzeichnet, daß** die Kristallisation in mehreren Stufen realisiert wird.

3. Verfahren nach einem der vorstehenden Ansprüche, **dadurch gekennzeichnet, daß** das Lactam ε-Caprolactam ist.

4. Verfahren nach Anspruch 3, **dadurch gekennzeichnet, daß** das ε-Caprolactam durch cyclisierende Hydrolyse von Aminocapronitril erzeugt wird.

5. Verfahren nach Anspruch 4, **dadurch gekennzeichnet, daß** das Aminocapronitril durch Hemihydrierung von Adiponitril synthetisiert wird.

6. Verfahren nach einem der vorstehenden Ansprüche, **dadurch gekennzeichnet, daß** das Lösungsmittel der Kristallisation aus der Gruppe gewählt wird, die ein Lactam, eine wäßrige Lösung von Lactam, Wasser; die gesättigten Kohlenwasserstoffe, die Alkohole, die aromatischen Kohlenwasserstoffe, die halogenierten organischen Verbindungen und die Ketone umfaßt.

7. Verfahren nach Anspruch 6, **dadurch gekennzeichnet, daß** das Lösungsmittel eine gesättigte wäßrige Lösung von Lactam ist.

8. Verfahren nach einem der vorstehenden Ansprüche, **dadurch gekennzeichnet, daß** die gewichtsmäßige Konzentration an Lactam in der zu kristallisierenden Lösung über 80 Gew.-% liegt.

## Claims

1. Process for purifying lactams obtained by cyclization of an aminoalkylnitrile, **characterized in that** it consists in subjecting the cyclization medium, consisting of the reaction medium obtained from the cyclization reaction, to a hydrogenation, in removing the volatile compounds and the high-boiling compounds, in treating the said medium with an ion-exchange resin and in recovering the lactam by crystallization.

2. Process according to Claim 1, **characterized in that** the crystallization is carried out in several steps.

3. Process according to either of the preceding claims, **characterized in that** the lactam is ε-caprolactam.

4. Process according to Claim 3, **characterized in that** the ε-caprolactam is produced by cyclizing hydrolysis of aminocapronitrile.

5. Process according to Claim 4, **characterized in that** the aminocapronitrile is synthesized by semihydrogenation of adiponitrile.

6. Process according to one of the preceding claims, **characterized in that** the crystallization solvent is chosen from the group comprising a lactam, an aqueous lactam solution, water, saturated hydrocarbons, alcohols, aromatic hydrocarbons, organohalogen compounds and ketones.

7. Process according to Claim 6, **characterized in that** the solvent is a saturated aqueous lactam solution.

8. Process according to one of the preceding claims, **characterized in that** the weight concentration of lactam in the solution to be crystallized is greater than 80% by weight.
